# EUROPEAN PATENT APPLICATION

(11) **EP 0 686 406 A1**
(43) Date of publication of application: **13.12.1995**
(21) Application number: 93910482.4
(22) Date of filing: 23.04.1993
(51) Int. Cl.: A61N 1/00, A61N 2/02, A61N 1/40

(54) **DEVICE FOR CHANGING THE ACTIVITY OF A BIOLOGICAL CELL**

(71) Applicant: PETRENKO, Sergei Ivanovich, Nizhny Novgorod, 603137 (RU)
(72) Inventor: PETRENKO, Sergei Ivanovich, Nizhny Novgorod, 603137 (RU)
(74) Representative: Lins, Edgar, Dipl.-Phys. Dr.jur.
(86) International application number: RU9300093
(87) International publication number: WO9425102

(57) **Abstract**

A device is disclosed for altering the activity of a biological cell, comprising: a receiver (1), an electromagnetic wave generator (3) and, connected to those components, a memory unit (2) designed as a single element (5) made of a solid or liquid material, and a temperature regulation unit (4) connected to the receiver (1), electromagnetic wave generator (3) and memory unit (2).

## Description

### Field of the Invention

This invention relates to medicine, namely, to medical equipment and refer to devices for changing the activity of a biological cell.

This invention may be useful for destruction of pathogen and opportunistic pathogen microorganisms in human and animal organisms .

### Description of the prior art

There is known the device for therapy with decimetric waves (USSR Inventor's Certificate # 942777, 1982, Int. Cl. A61 5/00) having a self-excited generator connected to a power meter through a control unit and a radiator of the contact type, series-connected to the power meter.

In device's operation mode, a signal from the self-excited generator defining the frequency of electromagnetic waves is supplied to a control unit providing amplification, control of electromagnetic power, and setting of the treatment procedure duration. Then the signal is applied to the power meter providing a dosed power level control and after that to the radiator which is in a direct with the patient.

Although this device provides for a higher precision for dosage of power absorbed by patient, it is incapable of acting selectively on specific types of biological cells.

There is known another device for therapy with low frequencies (USSR Inventor's Certificate No. 1581326, 1990, Int. Cl. A61 1/00) having a receiver of electromagnetic waves, generator of continuous electromagnetic waves, a generator of pulse electromagnetic waves, a switching unit, a radiator, an electrode, and a capacitor plate, said generators of electromagnetic waves being connected to the radiator, electrode, and capacitor plate through the switching unit.

Application of this device is based on correction of regulatory systems of human and animal organism by means of a weak low-frequency electromagnetic field.

This device operates in two main modes: in the diagnosis mode and in the therapy action mode. In the diagnosis mode the pathologic bioresonance frequency (or frequency spectrum) of a biological object is determined with the receiver, the generator of continuous electromagnetic waves, and the electrode.

The mode of action (distantly or locally) on a pathologic biological object by this device is set after determination of pathologic bioresonance frequency taking into account other medical indications. In this case, we set the forms of signals (pulse or continuous), amplitude, and waveform, therapeutic bioresonance frequency or frequency spectrum, duration of procedure .

The form of electromagnetic waves and duration of therapeutic procedure are selected by the switching unit which connects the generator of pulse or continuous waves to the radiator, the electrode or the capacitor plate.

Said device provides correction of functioning of biological cells by means of low-intensity electromagnetic waves.

And yet this device does not allow one to change gradually and selectively the activity of biological cells of the various types .

### Summary of the Invention

An object of this invention is to provide such a device for changing the activity of a biological cell that enables one to change cell activity of one type of cells without changing the activity of other types of cells due to resonance interaction between low intensive electromagnetic waves of this device and the cell on its bioresooance frequency.

Said object is accomplished due to the fact that a device for changing the activity of a biological cell having a receiver and a generator of electromagnetic waves located close to the cell, according to the invention, has a memory unit connected to the receiver and the generator of electromagnetic waves and a temperature control unit connected to the receiver, the generator of electromagnetic waves, and the memory unit, the receiver, the memory unit, and the generator of electromagnetic waves are unified into a single element made of a solid or liquid material.

This makes it possible to change the activity of one type of cells without changing the activity of other types of cells.

It is possible to use a chip as a solid or liquid substance of the single element.

This gives an opportunity to keep the device operable during a long time.

The temperature control unit may appear as a resistive element connected to a source of electric power.

This provides a wide range of temperature variation of the single element.

It is advisible to use the chip of a solid state device as a solid or liquid substance of the single element and to use at least one semiconductor junction of said device connected to the source of electric power as the temperature control unit.

This allows the overall dimensions of said device to be microminiaturized.

Used a solid state device may be a transistor having its emitter connected directly to the source of electric power and the collector connected to said source through a commutator with the base of said transistor connected to its collector through a resistor.

This allows one to simplify the device.

It is advisible to provide the device with a screened housing and a cover.

This protects information stored in the memory of the device against external effects.

The walls of the screened housing and the cover may be made of a water-containing salt. This provides for not only an effective screening, but also extends the range of ambient temperatures at which said device can operate.

The walls of the screened housing and the cover may be hollow.

This allows one to use liquids or loose substances as screening ones .

It is possible to fill the spaces in the walls of the screened housing and the cover with water.

This leads to higher efficiency of screening.

### Brief Description of the Drawings

Hereinafter this invention is explained by specific examples of its embodiment and the drawings attached thereto, wherein:
FIG.1 represents a general block fragrant of the device for changing the activity of a biological cell, according to the invention ;
FIG.2 shows a schematic diagram of one of the specific embodiments of the device for changing the activity of a biological cell, according to the invention;
FIG.3 illustrates a specific embodiment of the screened housing and the cover of the device for changing the activity of a biological cell, according to the invention.

### Detail Description of the Preffered Embodiment

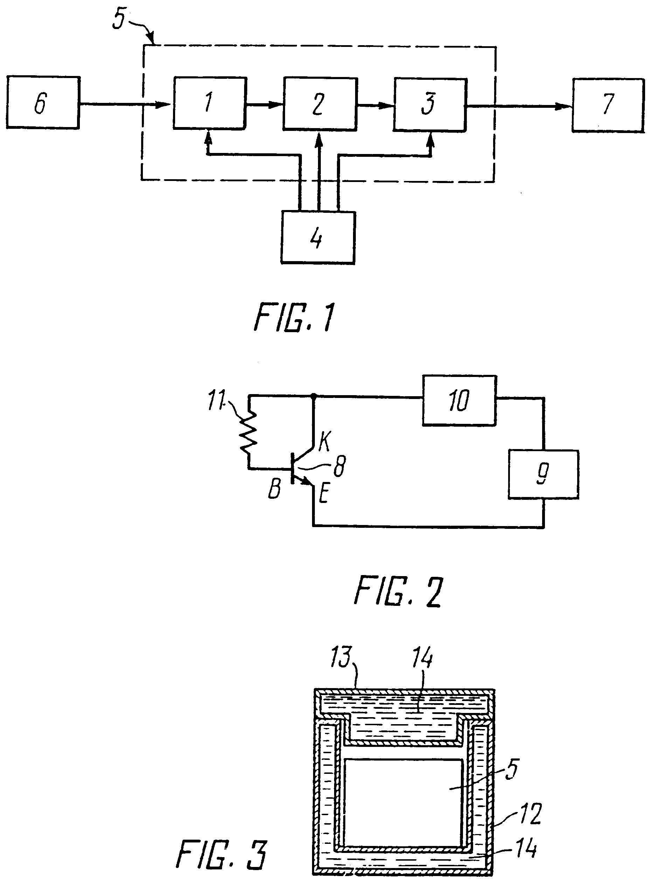

The device for changing the activity of a biological cell, as shown in FIG. 1, comprises a receiver 1, memory ttnit 2, and a generator 3 of electromagnetic waves which are electrically interconnected as well as a temperature control unit 4 connected to each of aforesaid components.

The receiver 1, the memory unit 2, and the generator 3 of electromagnetic waves may be interconnected in the various ways, their series connection will be described hereunder for the sake of easy understanding.

The receiver 1, the memory unit 2, and the generator 3 of electromagnetic waves are integrated into a single element 5 of a solid or liquid substance.

Used as a solid or liquid substance of the single element 5 may be any solids or liquid, e.g., copper, graphite, polyethylene, colophony, wood, water, etc. The substances having crystalline structure, high melting and boiling points, high resistance to the chemicals, low electric conductance, and a minimum content of impurities, for example, diamond, garnet, silicon, germanium, etc. can be used most efficiently as the single element 5.

The unit 4 for control of temperature of the sickle element 5 may be made directly as the source of of electric, mechanical, electromagnetic or any other energy. In this case the temperature of the single element 5 may be varied, for example, due to heat generation when electric current flows through said element, or due to heat liberation resulting from elastic strain of the single element 5, or due to to absorption of electromagnetic waves thereby, etc.

However, an element connected to the source of electric, chemical, mechanical, electromagnetic, etc. energy and placed in the immediate vicinity to the single element 5 or contacting with it may he most effectively used as the unit 4 for control of temperature.

The device for changing the activity of a biological cell operates as follows.

First, information on metabolic activity of reference cells is recorded into the memory unit 2 using any known technique .

For example, reference cells 6 of definite type having metabolic activity predetermined by any known technique are placed in the immediate vicinity to the single element 5. The single element 5 is heated by the unit 4 for control of temperature. In this case, an information on the metabolic activity of the reference cells 6 at the bioresonance frequency is recorded into the memory unit 5 using the receiver 1. The heating of the single element 5 may be followed by both forced or natural air cooling. The heating-and-cooling cycle of the single element 5 may be multiply repeated for example, in order to increase the level of information recording.

Heating followed by cooling or cooling followed by heating represent the cycle of temperature change of single element 5.

The device is ready to operate after the information on metabolic activity of the reference cells 6 has been recorded into the memory unit 2.
Next, a cell 7 whose biological activity should be changed (hereinafter referred to as irradiated cell ) is placed in the immediate vicinity to the single element 5 and the cycle of temperature change of the single element 5 is realized using the unit 4 for control of temperature. As the temperature of thesingle element 5 varies, the generator 3 radiates low intensivity electromagnetic waves on bioresonance freqnency of the reference cells 6.

As a consequence of resonance interaction of electromagnetic waves, radiated by the generator 3 of the single element 5 with the irradiated cell 7 at bioresonance frequency, the metabolic activity of the irradiated cell 7 is set similary to that of the reference cell 6.

Said recording and reproduction of information on metabolic activity of the reference cells 6 can be carried out by using the unit 4 for control of temperature, first, in the cooling mode of the single element 5, followed by its heating.

The chip of a solid-state device (FIG. 2) may be used as the single element 5 of the device. In this case, at least one semiconductor junction of said solid-state device 8, connected to the source of electric power, is used as the unit 4 for temperature control.

FIG. 2 represents an exemplary embodiment of the device, wherein a transistor is used as a solid state device, the transistor emitter E being connected directly to the source 9 of electric power, and a collector K is connected through a commutator 10 with the base B of the transistor 8 connected to its collector through a resistor 11.

The device shown in FIG. 2 operates as follows.

First, an information on metabolic activity of the reference cells 6 is recorded into the chip of the transistor 8, using any known technique.

For example, the reference cells 6 having specified metabolic activity determined by any known technique, are placed in the immediate vicinity to the transistor 8. The source 9 of electric energy is connected to the transistor 8 by closing the contacts of the commutator 10. The transistor chip is heated by heat generated at the semiconductor junctions of the transistor 8.The resistor 11 defines the mode of the transistor 8 and, correspondingly, the heating temperature of its chip. The recording of information on metabolic activity of the reference cells 6 into the chip of the transistor 8 is carried out when the chip is heated. Then the circuit of the source 9 of electric is disconnected by the commutator 10. Thus, the chip of the transistor 8 chip is cooled.

The information recording may also be done in the cooling mode of the chip of the transistor 8.

The device is ready for operation after having recorded of information on metabolic activity of the reference cells.

Then the irradiated cell 7 (or a group of cells) is placed in the immediate vicinity to the transistor 8 and the heating-and-cooling cycle of the chip of the transistor 8 is performed by closing and opening, respectively, the contacts of the commutator 10. As a result of resonanse interaction of low-intensity electromagnetic waves of the device and the irradiated cell 7 on the bioresonance frequency F, the metabolic activity of the irradiated cell is set similar to the activity of the reference cell.

In order to maintain the predetermined metabolic activity of irradiated cells over a long period of time, the repeated sessions of irradiation are carried out at definite time intervals.

In this case, the repeated recording of information into the device is not performed.

Now the operation of the device is considered by a specific example of changing the metabolic activity of bacterial cells of Staphylococcus aureus in human organism.

First, the cell culture of Staphylococcus aureus is taken, then it is set to the state with low metabolic activity by any known technique.

Thereupon,the information on low metabolic activity of the cells of Staphylococcus aureus is recorded by any known technique into the memory 2 of the single element 5. For example, the culture of these cells is placed in the immediate vicinity to the single element 5, and the heating-and-cooling cycle of the single element 5 with the use of the unit 4 for control of temperature is carried out.

Next, this device is placed in the immediate vicinity to the patient, and the heating- and-cooling cycle of the single element 5 is performed using the unit 4 for control of temperature. In this case, the resonance interaction of low intensity electromagnetic waves and the cells of Staphylococcus aureus in human organism occurs on the bioresonance frequency F. As a result, the cells of Staphylococcus aureus in human or animal organism are settled in the state of low metabolic activity similar to the activity of the reference cells of Staphlococcus aureus.

For maintaining over a long period of time the low metabolic activity of the cells Staphylococcus aureus located in human or animal organism and destruction of bacterial cells which are in the state of low metabolic activity by human immune system, it is necessary to perform several cycles of exposures, for example, the exposures are performed every 6 hours for 3 to 5 days.

The use of high-quality chips provides for prolonged storage and stable reproduction of recorded information. A drop in the level of information on metabolic activity of cell recorded in the chip of the transistor 8 is no more than 3% of the initial value after 1,000 cycles of heating and cooling of the chip of the transistor 8.

The device makes it possible to record and store in one chip the information on metabolic activity of the reference cells 6 having various bioresonance frequencies and to carry out concurrent action on similar types of cells on bioresocance frequencies F1, F2, F3,...,Fn respectively.
lo order to ensure the protection of information recorded in the device in the storage mode, the device is placed in a screened housing 12 with a cover 13 which protect the device against exposure to sources of high heat radiation, for example, burning spirit, gasoline, a match, a cigarette, etc.

A specific embodiment of the screened housing 12 and the cover 13 is shown in FIG. 3. The single element 5 and the unit 4 for control of temperature are placed inside the housing 12.

The housing 12 and the cover 13 can be made of a water-containing salt, for example, heptahydrate of magnesium sulfate MgSO₄ × 7H₂O, tetrahydrate of potassium-sodium tartrate KNC₄H₄O₆ × 4H₂O.

The walls of the housing 12 and the cover 13 may be made hollow, the water 14 can be placed into the space of the walls of housing 12 and the cover 13.

### Industrial Applicability

The proposed device can be used effectively for treatment of diseases caused by pathogen and opportunistic-pathogen microorganisms in human or animal organism.

The effect of the device in treatment of diseases caused by pathogen and opportunistic pathogen microorganisms is based on decreasing the rate of development thereby and making the immune system of a human or animal more active.

The decreasing of the rate of development of microorganisms is carried out by means of setting a potential on their cell membranes to a level, corresponding to their low metabolic activity.

An increase in the rate of destruction of microorganisms by human or animal immune system is performed automatically as the toxins emitted by bacteria are decreased due to decreasing of their metabolic activity.

The device can be used effectively for treatment, for example, of such diseases as pneumonia, angina, rheumatic arthritis, influenza, meningitis, etc.

The high selectivity and dosage of action, simplicity and operation reliability of the device along with its small dimensions ensure the conditions for use of the device for treatment of various human or animal diseases both in hospitals and ambulances .

The device has no contraindications to provide concurrent treatment by different methods.

## Claims

1. A device for changing the activity of a biological cell having a receiver and a generator of electromagnetic waves placed in the immediate vicinity to the cell, CHARACTERIZED in that it has a memory unit (2), connected to a receiver (1), and a generator (3) of electromagnetic waves, and a unit (4) for control of temperature, connected to the receiver (1), the generator (3) of electromagnetic waves, and the memory unit (2) with the receiver (1), the memory unit (2), and the generator (3) of electromagnetic waves being built up of the single element (5) of a solid or a liquid substance.

2. A device according to claim 1, CHARACTERIZED in that a chip is used as a solid or liquid substance of the single element (5).

3. A device according to claims 1, 2, CHARACTERIZED in that the unit (4) for control of temperature is built up of a resistive element connected to a source of electric power.

4. A device according to claims 2, 3, CHARACTERIZED in that the chip of a solid state device (8) is used as a solid or liquid substance of the single element (5), and at least one semiconductor junction of said device (8), connected to the source (9) of electric power, is used as the unit (4) for control of temperature.

5. A device according to claim 4, CHARACTERIZED in that a transistor, having an emitter (E) connected directly to the source of electric power (9) and a collector (K) connected to said source through a commutator (10) with a base (B) of said transistor connected to its collector (K) through a resistor (11), is used as the solid state device (8).

6. A device according to any said claims, CHARACTERIZED in that has a screened housing (12) and a cover (13).

7. A device according to claim 6, CHARACTERIZED in that the screened housing (12) and the cover (13) are built up of a salt which contains water.

8. A device according to claim 6, CHARACTERIZED in that the walls of the screened housing (12) and the cover (13) are made hollow.

9. A device according to claim 8, CHARACTERIZED in that the spaces in the walls of screened housing (12) and the cover (13) are filled with water (14).
